# EUROPEAN PATENT APPLICATION

(11) **EP 3 252 634 A1**
(43) Date of publication of application: **06.12.2017**
(21) Application number: 17171485.0
(22) Date of filing: 17.05.2017
(51) Int. Cl.: G06F 19/00, A61H 15/00, H04H 20/59

(54) **INFORMATION NETWORK SYSTEM INCLUDING MASSAGE MACHINE**

(30) Priority: 03.06.2016 JP 2016111869
(71) Applicant: Family Inada Co., Ltd., Osaka 532-0004 (JP)
(72) Inventor: INADA, Nichimu, Osaka, Osaka 532-0004 (JP); ISHIDOU, Yuta, Saihaku-gun, Tottori 689-3224 (JP); NOTSU, Yuji, Saihaku-gun, Tottori 689-3224 (JP); SASAKI, Izumi, Saihaku-gun, Tottori 689-3224 (JP)
(74) Representative: Horn Kleimann Waitzhofer Patentanwälte PartG mbB

(57) **Abstract**

Provided is an information network system in which a treatment subject can be informed that there is new information related to a massage machine when using the massage machine and the treatment subject can check and decide the information. There are provided a massage machine that has a treatment portion treating a treatment subject, a control unit controlling the treatment portion, and an operation unit for operating the treatment portion via the control unit; and a communication server that is connected to the control unit via a communication network. The communication server emits information related to the massage machine. The control unit is configured to issue a notification of reception of the information through a new arrival notification portion upon the reception of the information.

## Description

### TECHNICAL FIELD

The present invention relates to an information network system including a massage machine.

### BACKGROUND ART

In the related art, a treatment subject treats each site of the body by using a massage machine. In the massage machine, generally, a plurality of treatment courses are set in advance such that the treatment subject can make selection. Each of the treatment courses set in advance has a fixed treatment operation in which kneading, patting, and the like are combined.

The treatment subject often performs treatment by selecting a preferable treatment course from the plurality of treatment courses in accordance with the condition of the body. However, depending on the treatment subject, a relaxed state is sometimes affected in the treatment course set in advance, and there are cases where the massage machine is less likely to be used. Therefore, a massage machine in which the treatment course can be updated has been proposed.

For example, as such a technology in the related art, there is a technology in which after information regarding treatment contents is acquired through a portable communication terminal, data is downloaded through the portable communication terminal and is transferred to an operation portion of a massage machine so as to replace data in storage means such that an appropriate treatment state for a treatment subject is achieved (for example, refer to PTL 1).

In addition, as another technology in the related art, there is a technology in which a server prepares a massage course based on information from an external sensor such as a body composition meter, and data of the massage course is downloaded from the server via a communication portion of an operation unit for a massage machine and is executed (for example, refer to PTL 2).

### RELATED ART DOCUMENTS

### PATENT DOCUMENTS

Patent Document 1: JP-A-2014-39612
Patent Document 2: JP-A-2014-104178

### SUMMARY OF THE INVENTION

### PROBLEM THAT THE INVENTION IS TO SOLVE

However, in PTL 1, there is a need to obtain information regarding treatment contents through a portable communication terminal. Therefore, there is a need to download data through the portable communication terminal and to transfer the data to a massage machine through the portable communication terminal, thereby requiring a troublesome operation.

In addition, in PTL 2, a server prepares a course based on information from an external sensor such as a body composition meter. Therefore, a server which can conduct a complicated analysis is required. Moreover, the prepared massage course does not always have contents preferable for a treatment subject.

Furthermore, there are cases where treatment courses are revised from the contents at the time a massage machine is manufactured. However, generally, there are many cases where a massage machine is installed at home, and it is difficult to inform all of the massage machines of the presence of data to be updated.

An object of the present invention is to provide an information network system in which a treatment subject can be informed that there is newly-arrived information related to a massage machine when using the massage machine and the treatment subject can check and decide the information.

### [Means for solving the problem]

In order to achieve the object, according to the present invention, there is provided an information network system including a massage machine that has a treatment portion treating a treatment subject, a control unit controlling the treatment portion, and an operation unit for operating the treatment portion via the control unit; and a communication server that is connected to the control unit via a communication network. The communication server emits newly-arrived information related to the massage machine. The control unit is configured to issue a notification of reception of the newly-arrived information upon reception of the newly-arrived information through a new arrival notification portion. In the documents of this specification and Claims, examples of the "newly-arrived information" include information related to a treatment course and the like of the massage machine, information related to a program of the massage machine, and information related to music and the like. In addition, the "new arrival notification portion" can be an independent new arrival notification portion, a part of the massage machine, or the like, in addition to the operation unit. Examples of the independent new arrival notification portion include a smart phone and a tablet terminal. Examples of the "notification" include a notification given through a voice and a notification given through light, in addition to a notification given through screen display.

According to the configuration, when new information related to the massage machine is transmitted from the communication server, the massage machine can cause a new arrival display portion to display the received new information and can inform the treatment subject of the presence of the new information. The treatment subject can check that the new information has been delivered, through the new arrival display portion when using the massage machine.

In addition, the communication server may emit update information related to the treatment course of the massage machine to the control unit. The control unit may be configured to notify an information notification portion of the update information and to download and introduce update data of the treatment course from the communication server when the update information is decided. Examples of the "information notification portion" include an independent information notification portion (for example, a smart phone and a tablet terminal), in addition to the operation unit. Examples of "decision of the update information" include selection of "decide" displayed in a screen, an operation of pressing a decision button, and an operation performed through voice recognition. Examples of "introduction" include installation of the update data.

According to such a configuration, when there is a need to update the treatment course included in the massage machine, the treatment subject can be notified through the information notification portion. When the treatment subject decides to perform updating, data related to the updated treatment course is downloaded and is updated via a network. Thus, the treatment subject can make the treatment course of the massage machine to be in a latest state at all times by directly making selection. In addition, it is also possible to be informed of the update information such as addition of a function related to the treatment course via the network.

In addition, the communication server may emit information related to a new treatment course of the massage machine to the control unit. The control unit may be configured to notify the information notification portion of the information related to a new treatment course and to download and introduce data of the new treatment course from the communication server when the new treatment course is decided to be introduced. Examples of "decision of introduction" include selection of "decide" displayed in the screen, an operation of pressing the decision button, and an operation performed through voice recognition.

According to such a configuration, the treatment subject can be informed of the new treatment course through the information notification portion. When the treatment subject decides to introduce the new treatment course, the data of the new treatment course is downloaded and introduced via the network. For example, when the treatment subject decides to introduce the new treatment course, the data of the new treatment course is sent to the control unit of the massage machine via the network and can be installed by the control unit. Thus, the treatment subject can immediately use the new treatment course. In addition, the information related to a new treatment course can also be informed via the network.

In addition, the communication server may emit update information related to a system program of the massage machine to the control unit. The control unit may be configured to download and introduce update data of the system program from the communication server.

According to such a configuration, the information related to the system program of the massage machine is transmitted to the control unit from the communication server. When there is a need to perform updating, the control unit can download and update the update data via the network. Thus, the massage machine can update the system program. For example, enhancement of security and bugs of the system program can be automatically revised.

In addition, a billing system may be configured to charge the treatment subject for the cost when at least one of decision of the updating, decision of introducing the new treatment course, updating related to the system program, and purchase of merchandise notified through the new arrival notification portion is decided. The "billing system" indicates a system in which a service provider or an external service provider managing the communication server charges the treatment subject for the cost.

According to such a configuration, when any one of decision of updating, decision of introducing the new treatment course, updating related to the system program, and purchase of merchandise notified through the new arrival notification portion is decided, the billing system charges the treatment subject. Therefore, the treatment subject can easily pay for the cost of updating data and the like in the massage machine. As the billing system, a fee collecting system or the like of a communication service provider can be used.

In addition, the operation unit may further have a function as an information instrument performing information communication with the communication server via the control unit, and a screen for displaying information. The communication server may emit information to the control unit. The operation unit may have a selection portion selecting necessary information from the information and causing the screen to display the selected information.

According to such a configuration, the treatment subject can perform treatment while seeing the information emitted from the communication server when the massage machine is in use. Examples of the information can include new music, advice related to health, introduction of health equipment, and introduction of health food. The treatment subject selects the information and can see the selected information displayed in the screen of the operation unit when the massage machine is in use.

In addition, the massage machine may have positional information. The control unit may transmit the positional information to the communication server. The communication server may transmit an emergency alert corresponding to the positional information or an emergency alert independent from the positional information to the control unit. The control unit may be configured to issue a notification of the received emergency alert through an emergency alert notification portion. Examples of the "emergency alert notification portion" include an independent emergency alert notification portion and a part of the massage machine, in addition to the operation unit. Examples of the "notification" include a notification given through a voice and a notification given through light, in addition to a notification given through screen display.

According to such a configuration, the treatment subject can easily check that the emergency alert is emitted during treatment.

In addition, the control unit may be configured to stop the treatment portion upon reception of the emergency alert and to return to an initial state. Examples of "stopping the treatment portion" include stopping a kneading ball, and compressing/releasing an air bag. The "initial state" indicates a state at the time the treatment subject starts a massage.

According to such a configuration, the massage machine returns to a state at the time treatment starts, upon reception of the emergency alert. Therefore, the treatment subject can easily perform an action thereafter. For example, in a case of a chair-type massage machine, the massage machine returns to a state where the treatment subject is seated in the seat portion at the time before treatment starts. Therefore, the treatment subject is in a state of being likely to act.

In addition, the massage machine may have at least a seat portion in which the treatment subject takes a seat, and a backrest portion which supports the upper half of the body of the treatment subject who takes a seat in the seat portion, from the back. The control unit may be configured to erect at least the backrest portion upon reception of the emergency alert.

According to such a configuration, the backrest portion is erected upon reception of the emergency alert. Therefore, the treatment subject can be in a state of being likely to act.

### ADVANTAGE OF THE INVENTION

According to the present invention, a treatment subject can be informed that there is newly-arrived information related to the massage machine when using the massage machine. The treatment subject checks information such as updating the treatment course related to the massage machine, and a countermeasure can be decided upon the determination of the treatment subject.

### BRIEF DESCRIPTION OF THE DRAWINGS

- Fig. 1: is the overall configuration diagram illustrating an information network system according to an embodiment of the present invention.
- Fig. 2: is a perspective view of a massage machine illustrated in Fig. 1.
- Fig. 3: is a functional block diagram of the massage machine illustrated in Fig. 2.
- Fig. 4: is a front view illustrating an example of a screen of an operation unit illustrated in Fig. 2.
- Fig. 5: is a front view illustrating an example of a newly-arrived notification in the screen of the operation unit illustrated in Fig. 4.
- Fig. 6: is a configuration diagram of an example in which a billing system is included in the information network system illustrated in Fig. 1.
- Fig. 7: is a front view of the screen illustrating an example of displaying information in the operation unit illustrated in Fig. 4.
- Fig. 8: is a front view illustrating an example of displaying the screen in which information in the operation unit illustrated in Fig. 7 is selected.

### BEST MODE FOR CARRYING OUT THE INVENTION

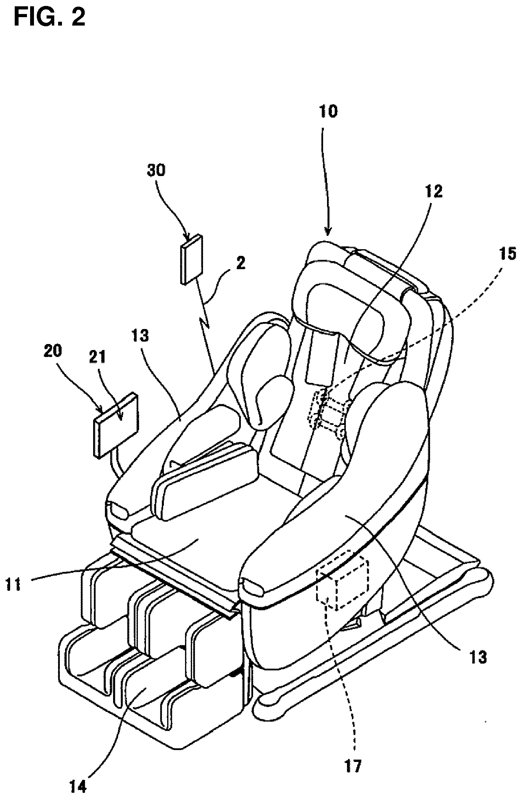

Hereinafter, an embodiment of the present invention will be described based on the drawings. The concept of forward, rearward, leftward, and rightward directions in the documents of this specification and Claims coincides with the concept of directions viewed from a treatment subject when the treatment subject takes a seat in a seat portion 11 of a massage machine 10 illustrated in Fig. 2.

### Configuration of Information Network System

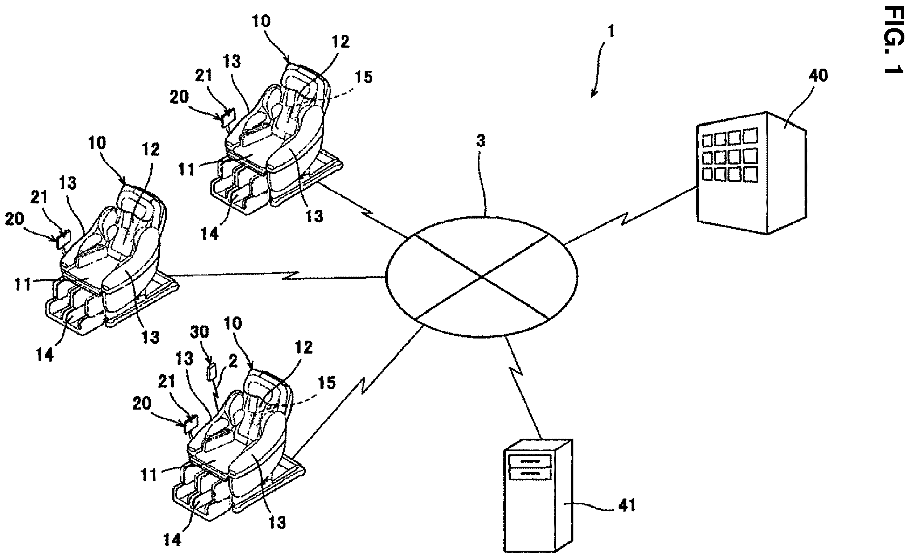

As illustrated in Fig. 1, an information network system 1 including the massage machine 10 of the embodiment includes the massage machine 10 treating the treatment subject, a service provider 40, and a communication server 41 within a communication network 3. Examples of the service provider 40 include a service provider 40 providing rental service of the massage machine 10, and a service provider 40 managing the massage machine 10 which has been sold. The communication server 41 is configured to be able to communicate with the massage machine 10 via the communication network 3. Examples of the communication network 3 include the internet network, a communication network in a building, and a local network. In this embodiment, an example in which the service provider 40 has a cloud-type communication server 41 will be described. The communication server 41 may be a communication server 41 managed by the service provider 40 or may be a communication server 41 managed by a different service provider 40. The communication server 41 emits information related to the massage machine 10.

### Configuration of Massage Machine

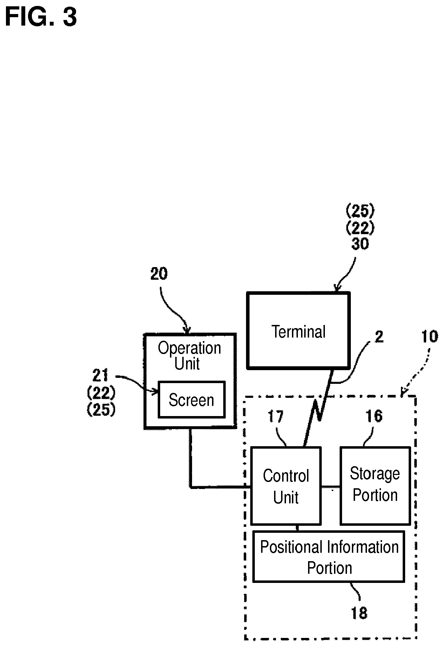

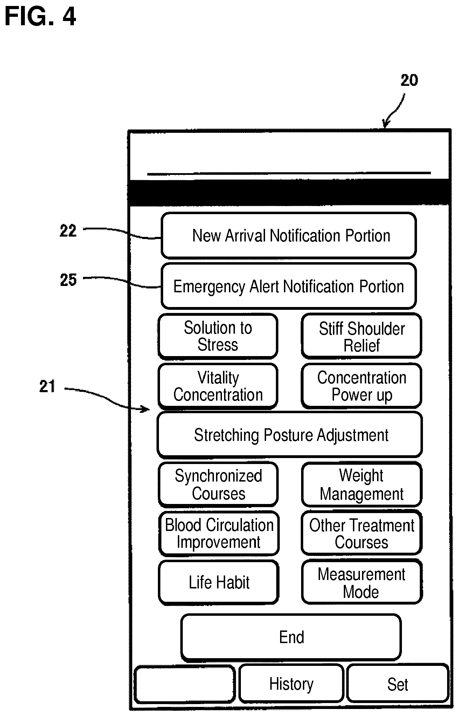

Fig. 2 is a perspective view of the massage machine 10, and Fig. 3 is a functional block diagram of the massage machine 10. Fig. 4 is a front view illustrating an example of a screen of an operation unit illustrated in Fig. 2. As illustrated, the massage machine 10 has the seat portion 11 in which the treatment subject takes a seat, and a backrest portion 12 which supports the upper half of the body of the treatment subject who takes a seat in the seat portion 11, from the back. The seat portion 11 has armrests 13 at the right and left positions. The seat portion 11 has a footrest 14 in the front of the seat portion 11. In this example, a treatment portion (kneading ball unit) 15 of the backrest portion 12 is illustrated. Other portions are also provided with a treatment portion such as an air bag. The treatment portion 15 is controlled by a control unit 17. A plurality of treatment courses (will be described later) are set from combinations of the treatment portion 15 and are stored in a storage portion 16.

In a portion of the armrest 13, there is provided an operation unit 20 for operating the massage machine 10. The operation unit 20 has a screen 21. A treatment operation of the treatment portion 15 or the like is operated by using the operation unit 20. The operation unit 20 is provided so as to be able to be operated in a state where the treatment subject takes a seat. The operation unit 20 can be an operation unit 20 such as a tablet terminal. The control unit 17 is connected to the operation unit 20. The screen 21 of the operation unit 20 functions as a new arrival notification portion 22 notifying the treatment subject of newly-arrived information related to the massage machine 10.

When the control unit 17 of the massage machine 10 receives information emitted from the communication server 41, for example, the control unit 17 issues a notification regarding the information which is received through the new arrival notification portion 22 set in a part of the screen 21 of the operation unit 20. Examples of the notification include a notification through a voice from a speaker, and a notification through light such as an LED, in addition to a notification through screen display.

In addition, in addition to the operation unit 20, the massage machine 10 of the embodiment includes a terminal 30 having the new arrival notification portion 22. The terminal 30 may have a screen, may have a speaker for issuing a notification of a new arrival, and may have an LED for issuing a notification of a new arrival. The terminal 30 can communicate with the massage machine 10 through radio communication (BLUETOOTH (registered trademark)) 2. The terminal 30 may be connected to the massage machine 10 by radio or through a cable of different type. Moreover, the new arrival notification portion 22 can serve as an emergency alert notification portion 25 when an emergency alert is received (will be described later). Such a "new arrival notification portion 22" can be an independent new arrival notification portion 22 (terminal 30 or the like) and a part of the massage machine, in addition to the operation unit 20. Examples of the independent new arrival notification portion 22 include a smart phone and a tablet terminal.

The massage machine 10 is provided with the control unit 17 which controls an operation of the treatment portion 15, an erect/tilt angle of the backrest portion 12 according to the treatment course (will be described later), and the like; and the storage portion 16 which stores the plurality of treatment courses having treatment contents for the treatment portion 15 different from each other. For example, the control unit 17 has a CPU, a ROM, a RAM, and the like. For example, as the storage portion 16, a flash memory, a hard disk drive, or the like can be used.

In this manner, when new information related to the massage machine 10 is transmitted from the communication server 41, the massage machine 10 causes the new arrival notification portion 22 to display the received new information, thereby informing the treatment subject of the presence of the new information. The treatment subject can check that the new information has been delivered, through the new arrival notification portion 22 when using the massage machine 10.

In addition, the massage machine 10 has a positional information portion 18. As positional information of the massage machine 10, a signal is sent from the positional information portion 18 of the massage machine 10 to the communication server 41, and a position (region) is specified through the communication server 41. The position (region) of the massage machine 10 can be specified through GPS, region discrimination means or the like using communication means in use (for example, communication means such as WI-FI and LTE). Accordingly, as described below, when an emergency alert is emitted in the region where the massage machine 10 is positioned, the emergency alert is transmitted from the communication server 41 to the massage machine 10 and is informed to the treatment subject through the emergency alert notification portion 25.

### Example of Newly-arrived Information related to Massage Machine

Examples of the newly-arrived information related to the massage machine 10 include update information related to the treatment course, information related to a new treatment course, update information related to a system program, and information related to new music. The newly-arrived information is not limited to the examples.

### Update Information related to Treatment Course

The communication server 41 emits the update information related to the treatment course of the massage machine 10 to the control unit 17. The control unit 17 issues a notification regarding the presence of the newly-arrived information through the new arrival notification portion 22 of the operation unit 20. The notification through the new arrival notification portion 22 may be set such that the treatment subject selects the new arrival notification portion 22 as in this example. However, without being selected by the treatment subject, contents of the update information can be notified through an information notification portion 23. In this example, when the new arrival notification portion 22 is selected by the treatment subject, a notification regarding the presence of the update information is issued through the information notification portion 23 of the screen 21 of the operation unit 20. Examples of the notification include a notification through screen display, voice guidance, and flashing of light. In addition, it is also possible to be informed of the update information such as addition of a function related to the treatment course via the communication network 3. The treatment subject can determine whether or not to update the treatment course. When the treatment subject decides to perform updating, update data of the treatment course is downloaded from the communication server 41 via the communication network 3. The update data is installed by the control unit 17, and the treatment course is updated. Accordingly, the treatment course of the massage machine 10 can be in a latest state.

In this manner, when there is a need to update the treatment course included in the massage machine 10, the treatment subject can be notified through the information notification portion 23. The treatment subject can decide whether or not to perform updating upon his/her own determination.

### Information related to New Treatment Course

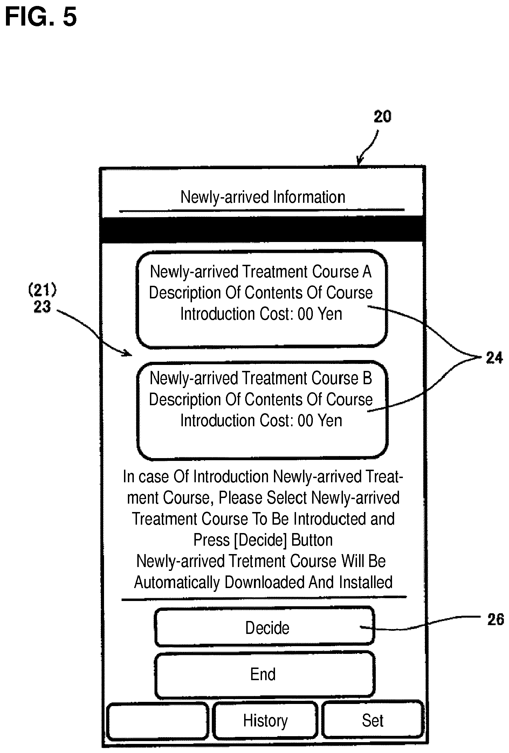

Fig. 5 is a front view illustrating an example of a newly-arrived notification in the screen 21 of the operation unit 20. The communication server 41 emits the information related to a new treatment course of the massage machine 10 to the control unit 17. The control unit 17 issues a notification regarding the presence of the newly-arrived information through the new arrival notification portion 22 of the operation unit 20. In this case as well, the notification through the new arrival notification portion 22 may be set such that the treatment subject selects the new arrival notification portion 22 as in this example. However, without being selected by the treatment subject, contents of the new treatment course can be notified through the information notification portion 23. In this example, when the new arrival notification portion 22 is selected by the treatment subject, a notification regarding the presence of the information related to a new treatment course is issued through the information notification portion 23 of the screen 21 of the operation unit 20. In addition, the information related to a new treatment course can also be informed via the communication network 3. In this example, it is possible to be informed that there are "newly-arrived treatment course A" and "newly-arrived treatment course B". In addition, the contents related to each of the newly-arrived treatment courses and the introduction cost can be checked. The treatment subject can check the information and can determined whether or not to introduce the new treatment course. When the treatment subject selects the new treatment course to be introduced and presses a [decide] button 26, data of the new treatment course is downloaded from the communication server 41 via the communication network 3. The data of the new treatment course is installed by the control unit 17. Accordingly, the treatment subject can immediately use the new treatment course.

In this manner, the new treatment course of the massage machine 10 can be informed to the treatment subject by issuing a notification through the new arrival notification portion 22. The treatment subject can decide whether or not to introduce the new treatment course upon his/her own determination by causing the information notification portion 23 to display the new treatment course of the newly-arrived information and checking the new treatment course.

Similar to the information related to a new treatment course, the information related to new music is also emitted from the communication server 41 to the massage machine 10 and can be notified through the new arrival notification portion 22 of the massage machine 10. The treatment subject can also decide whether or not to introduce new music (merchandise) upon his/her own determination. In addition, examples of the newly-arrived information notified through the new arrival notification portion 22 can include information related to merchandise such as new health food. The treatment subject checks the newly-arrived information through the information notification portion 23 and can decide purchase of the merchandise upon his/her own determination.

### Update Information related to System Program

The communication server 41 emits the update information related to the system program of the massage machine 10 to the control unit 17. In a case of the update data of the system program, the control unit 17 can automatically download and introduce the update data from the communication server 41. In addition, the control unit 17 issues a notification through the new arrival notification portion 22 such that the system program is to be updated.

In this manner, when the information is emitted from the communication server 41 to the control unit 17 regarding the system program of the massage machine 10, if there is a need to perform updating, the control unit 17 can download the update data via the communication network 3 and can perform updating. Accordingly, regarding the system program of the massage machine 10, for example, updating can be performed such that enhancement of security and bugs of the system program are automatically revised.

In this embodiment, description is given regarding an example in which the update information, the information related to a new treatment course, and the like are notified through the information notification portion 23 of the operation unit 20. However, in a case of including the terminal 30 having the new arrival notification portion 22, the terminal 30 may be caused to be provided with the information notification portion 23 such that the update information, the information related to a new treatment course, and the like are notified through the terminal 30.

### Configuration of Billing System

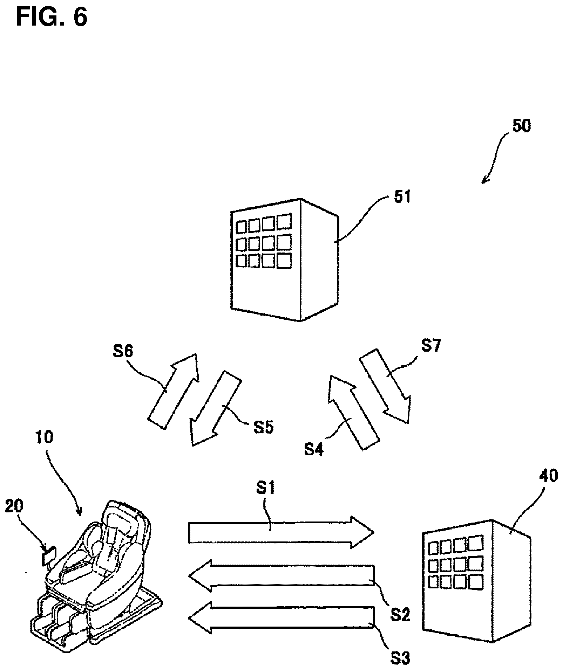

Fig. 6 is a configuration diagram of an example in which a billing system 50 is included in the information network system 1. As illustrated, in addition to the treatment subject using the massage machine 10, and the service provider 40, a communication service provider 51 managing the billing system 50 is included. The billing system 50 indicates a system of charging the treatment subject for the cost. In a case of this example, as the billing system 50, a fee collecting system or the like of the communication service provider 51 can be used. In the illustrated example, when the treatment subject decides the purchase (S1), the service provider 40 checks the treatment subject for the purchase (S2) and transmits data (S3). Thereafter, the service provider 40 charges the communication service provider 51 for the cost (S4). The communication service provider 51 charges the treatment subject for the purchase cost together with the communication cost and the like with respect to the service provider 40 (S5). The treatment subject makes payment with respect to the communication service provider 51 (S6). Then, the purchase cost of the treatment subject is payed from the communication service provider 51 to the service provider 40 (S7).

In this manner, the treatment subject decides at least one of decision of updating, decision of introducing the new treatment course, updating related to the system program, and purchase of merchandise notified through the new arrival notification portion 22, the billing system 50 charges the treatment subject for the cost. In a case where the cost occurs due to updating the treatment course, introducing a new treatment course, updating the system program, or the like, the billing system 50 charges the treatment subject for the cost. Therefore, the cost can be easily paid.

As the billing system 50, in addition to the service provider managing the communication server 41 and an external service provider, the service provider 40 may have the billing system 50.

### Configuration as Information Instrument

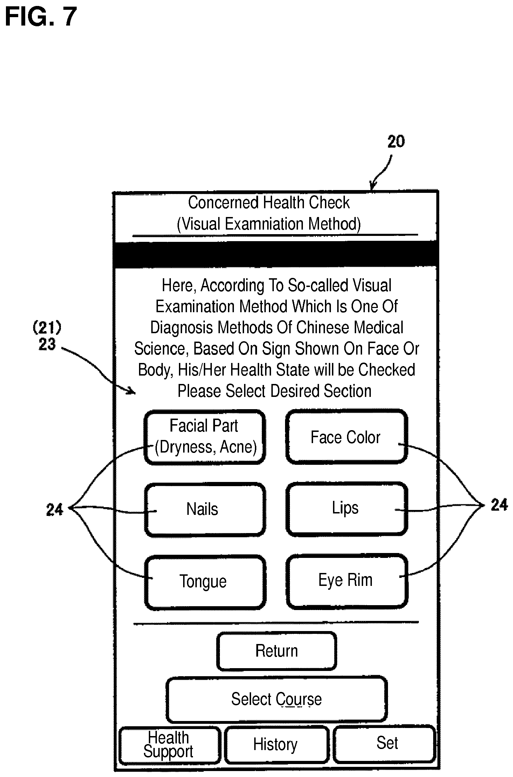

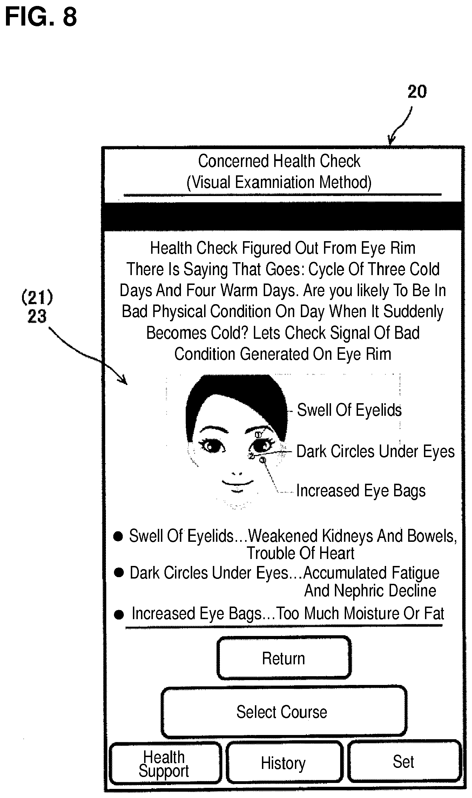

Fig. 7 is a front view of the screen 21 illustrating an example of displaying information in the operation unit 20. Fig. 8 is a front view illustrating an example of displaying the screen in which information in the operation unit 20 illustrated in Fig. 7 is selected. Based on the drawings, description will be given regarding an example in which information is transmitted from the communication server 41 to the massage machine 10 and is displayed in the operation unit 20. In this embodiment, the operation unit 20 further includes a function as an information instrument performing information communication with the communication server 41 via the control unit 17, and the screen 21 displaying information. The screen 21 is the same.

The communication server 41 emits the newly-arrived information to the control unit 17. The control unit 17 issues a notification through the new arrival notification portion 22 of the operation unit 20 regarding the presence of the newly-arrived information. When the treatment subject selects the new arrival notification portion 22 of the operation unit 20, the screen 21 of the operation unit 20 displays the newly-arrived information. The treatment subject can select necessary information through selection portions 24 from the newly-arrived information displayed in the screen 21. In this example, description will be given regarding a case where "concerned health check (visual examination method)" is selected.

As illustrated in Fig. 8, when selection (decision) is made through the operation unit 20, the screen for "concerned health check (visual examination method)" is displayed. In this example, the health check can be performed for "facial part", "face color", "nails", "lips", "tongue", and "eye rim".

For example, when "eye rim" is selected, the screen illustrated in Fig. 7 is displayed. As the "health check figured out from the eye rim", a comment is displayed. In this example, "There is a saying that goes: a cycle of three cold days and four warm days. Aren't you likely to be in bad physical condition on a day when it suddenly becomes cold? Let's check a signal of bad condition generated on the eye rim" is displayed. A comment based on the state of the eye rim is displayed. In this example, "swell of eyelids ... weakened kidneys and bowels, trouble of the heart", "dark circles under eyes ... accumulated fatigue and nephric decline", "increased eye bags ... too much moisture or fat", and the like are displayed. Description related to other health checks will be omitted. In addition, the above-referenced items for health check are examples and a different health check can be adopted.

In this manner, the treatment subject can perform treatment while seeing the information emitted from the communication server 41 when the massage machine 10 is in use. Examples of the information can include new music, advice related to health, introduction of health equipment, and introduction of health food, in addition to information related to health. The treatment subject selects the information and can see various types of information displayed in the screen 21 of the operation unit 20 when the massage machine 10 is in use. According to such a function, the treatment subject can perform treatment while being relaxed.

### Configuration of Receiving Emergency Alert

The massage machine 10 of the embodiment has the positional information portion 18 acquired through GPS. The communication server 41 has information for specifying a region in which the massage machine 10 is positioned, through the positional information portion 18 of the massage machine 10. As described above, as the information for specifying the position (region) of the massage machine 10, the region discrimination means or the like using the communication means in use (for example, the communication means such as WI-FI and LTE) can be used in addition to GPS.

When an emergency alert for the region corresponding to the positional information of the massage machine 10 is emitted, the communication server 41 transmits the emergency information to the control unit 17. The control unit 17 of the massage machine 10 notifies the treatment subject of the received emergency alert through the emergency alert notification portion 25. In addition, as necessary, an emergency alert independent from the positional information is also transmitted from the communication server 41 to the control unit 17, and the received emergency alert may be notified to the treatment subject through the emergency alert notification portion 25.

The emergency alert notification portion 25 may be a part of the screen 21 of the operation unit 20. In addition, in this embodiment, the emergency alert notification portion 25 can be the terminal 30 other than the operation unit 20. Examples of the emergency alert notification portion 25 include a portion independent from the massage machine 10, and a part of the massage machine.

Moreover, Examples of the notification of the emergency alert can include a notification through a voice, and a notification through light, in addition to a notification through screen display. For example, a notification through a voice such as "Pleas immediately check the emergency alert which has been received" can be combined with flashing red light. According to such a configuration, the treatment subject can be easily informed that an emergency alert is emitted during treatment.

In addition, the control unit 17 can stop the operation of the treatment portion 15 upon reception of the emergency alert and can cause the treatment portion 15 to return to the initial state. Examples of stopping an operation of the treatment portion 15 include stopping a kneading ball, and compressing/releasing an air bag. In this manner, the massage machine 10 automatically returns to a state at the time treatment starts, upon reception of an emergency alert. Therefore, the treatment subject can easily perform an action thereafter.

Moreover, the massage machine 10 of the embodiment is a chair-type massage machine 10 having the seat portion 11 in which the treatment subject takes a seat, and the backrest portion 12 which supports the upper half of the body of the treatment subject who takes a seat in the seat portion 11, from the back. Therefore, the control unit 17 can erect at least the backrest portion 12 upon reception of an emergency alert. In this manner, in a case of the chair-type massage machine 10, upon reception of an emergency alert, the massage machine 10 returns to a state where the treatment subject is seated in the seat portion 11 at the time before treatment starts. Therefore, the treatment subject is in a state of being likely to perform action thereafter.

### General

As described above, according to the information network system 1 including the massage machine 10, the treatment subject can be informed that there is the newly-arrived information related to the massage machine 10 when using the massage machine 10. The treatment subject checks updating the treatment course related to the massage machine 10, information of a new treatment course, and the like and a countermeasure can be decided upon the determination of the treatment subject. Accordingly, the function of the massage machine 10 can be appropriately retained via the communication network 3.

Moreover, improvement and the like of the function of the massage machine 10 can be achieved upon the determination of the treatment subject. Therefore, the massage machine 10 can be in a state desired by the treatment subject.

In addition, in a case where an emergency alert is emitted, the massage machine 10 can be controlled such that the treatment subject can easily act, and the treatment subject can easily act at the time of emergency.

In the embodiment described above, description has been given regarding an example of the massage machine 10 which has the seat portion 11 and the backrest portion 12 and in which the backrest portion 12 can be erected and tilted. However, the embodiment can also be applied to a massage machine in which the angle of the backrest portion 12 is fixed, and a massage machine other than the chair-type massage machine. The type of the massage machine 10 is not limited to the embodiment described above.

In addition, in the embodiment described above, description has been given regarding an example in which the massage machine 10, the communication server 41, and the service provider 40 are connected together via the communication network 3. However, the embodiment may have a configuration in which the communication service provider 51 operating the billing system 50 is also connected to the communication network 3. The information network system 1 is not limited to the configuration of the embodiment.

Moreover, the embodiment described above is an example, and the communication server 41 may be integrated with the service provider 40 or may be a server managed by a different service provider. Various changes can be made within the scope not harming the gist of the present invention, and the present invention is not limited to the embodiment described above.

### LIST OF REFERENCE NUMERALS

1 INFORMATION NETWORK SYSTEM
2 RADIO COMMUNICATION (BLUETOOTH (REGISTERED TRADEMARK))
3 COMMUNICATION NETWORK
10 MASSAGE MACHINE
11 SEAT PORTION
12 BACKREST PORTION
15 TREATMENT PORTION (KNEADING BALL UNIT)
16 STORAGE PORTION
17 CONTROL UNIT
18 POSITIONAL INFORMATION PORTION
20 OPERATION UNIT
21 SCREEN
22 NEW ARRIVAL NOTIFICATION PORTION
23 INFORMATION NOTIFICATION PORTION
24 SELECTION PORTION
25 EMERGENCY ALERT NOTIFICATION PORTION
30 TERMINAL
40 SERVICE PROVIDER
41 COMMUNICATION SERVER
50 BILLING SYSTEM
51 COMMUNICATION SERVICE PROVIDER

## Claims

1. An information network system including a massage machine, comprising:
a massage machine that has a treatment portion treating a treatment subject, a control unit controlling the treatment portion, and an operation unit for operating the treatment portion via the control unit; and
a communication server that is connected to the control unit via a communication network,
wherein the communication server emits information related to the massage machine, and
wherein the control unit is configured to issue a notification of reception of the information through a new arrival notification portion upon the reception of the information.

2. The information network system including a massage machine according to Claim 1,
wherein the communication server emits update information related to a treatment course of the massage machine to the control unit, and
wherein the control unit is configured to notify an information notification portion of the update information and to download and introduce update data of the treatment course from the communication server when the update information is decided.

3. The information network system including a massage machine according to Claim 1 or 2,
wherein the communication server emits information related to a new treatment course of the massage machine to the control unit,
wherein the control unit is configured to notify the information notification portion of the information related to a new treatment course and to download and introduce data of the new treatment course from the communication server when the new treatment course is decided to be introduced.

4. The information network system including a massage machine according to any one of Claim 1 to 3,
wherein the communication server emits update information related to a system program of the massage machine to the control unit, and
wherein the control unit is configured to download and introduce update data of the system program from the communication server.

5. The information network system including a massage machine according to any one of Claim 2 to 4,
wherein a billing system is configured to charge the treatment subject for the cost when at least one of decision of the updating, decision of introducing the new treatment course, updating related to the system program, and purchase of merchandise notified through the new arrival notification portion is decided.

6. The information network system including a massage machine according to any one of Claim 1 to 5,
wherein the operation unit further has a function as an information instrument performing information communication with the communication server via the control unit, and a screen for displaying information,
wherein the communication server emits information to the control unit, and
wherein the operation unit has a selection portion selecting necessary information from the information and causing the screen to display the selected information.

7. The information network system including a massage machine according to any one of Claim 1 to 6,
wherein the massage machine has a positional information,
wherein the control unit transmits the positional information to the communication server,
wherein the communication server transmits an emergency alert corresponding to the positional information or an emergency alert independent from the positional information to the control unit, and
wherein the control unit is configured to issue a notification of the received emergency alert through an emergency alert notification portion.

8. The information network system including a massage machine according to Claim 7,
wherein the control unit is configured to stop the treatment portion upon reception of the emergency alert and to return to an initial state.

9. The information network system including a massage machine according to Claim 7 or 8,
wherein the massage machine has at least a seat portion in which the treatment subject takes a seat, and a backrest portion which supports the upper half of the body of the treatment subject who takes a seat in the seat portion, from the back, and
wherein the control unit is configured to erect at least the backrest portion upon reception of the emergency alert.
